# EUROPÄISCHE PATENTSCHRIFT

(11) **EP 0 633 019 B1**
(45) Veröffentlichungstag und Bekanntmachung des Hinweises auf die Patenterteilung: **11.08.1999**
(21) Anmeldenummer: 94107874.3
(22) Anmeldetag: 21.05.1994
(51) Int. Cl.: A61K 9/00

(54) **Druckgaspackungen unter Verwendung von Polyoxyethylen-glyceryl-fettsäure-estern als Suspensionsstabilisatoren und Ventilschmiermittel**
Pressurized gas packagings using polyoxyethylene glyceryl fatty acid esters as suspension stabilizers and valve lubricants
Conditionnement de gaz comprimé avec polyoxyéthylène glyceryl esters d'acide gras comme stabilisateurs de suspensions et lubricants pour valves

(30) Priorität: 08.07.1993 DE 4322703
(43) Veröffentlichungstag der Anmeldung: 11.01.1995
(73) Patentinhaber: ASTA Medica Aktiengesellschaft, 01277 Dresden (DE)
(72) Erfinder: Hettche, Helmut, Dr., D-63128 Dietzenbach (DE); Muckenschnabel, Reinhard, Dr., D-60388 Frankfurt (DE)
(74) Vertreter: Decker, Karl-Ludwig

(56) Entgegenhaltungen:
- EP-A- 0 206 291
- WO-A-93/18746
- US-A- 5 156 833

## Beschreibung

Aerosol-Druckgaspackungen werden seit vielen Jahren für die unterschiedlichsten Zwecke verwendet. Unter Aerosol-Druckgaspackungen versteht man druckfeste Behältnissse, aus denen eine unter Druck stehende Mischung aus verflüssigtem Treibgas und Wirkstoff durch Betätigung eines Ventils freigesetzt wird. Druckgaspackungen sind beispielsweise in Sucker, Fuchs und Speiser (Herausgeber), Pharmazeutische Technologie, Thieme, Stuttgart, 1991, S. 673 - 688 beschrieben, weiter werden Aerosole und Druckgaspackungen in List, Arzneiformenlehre, Wissenschaftliche Verlagsgesellschaft, Stuttgart, 1985, S. 8 - 18 und in Voigt, Lehrbuch der pharmazeutischen Technologie, VCh, Weinheim, 1987 auf den Seiten 427 - 436 beschrieben. Weiter wird diese populäre Darreichungsform von Thoma, Aerosole, Selbstverlag, Frankfurt am Main, 1979 ausführlich erläutert. Im medizinischen Bereich werden sie vorteilhaft dann eingesetzt, wenn Wirkstoffe direkt in die Lunge transportiert und dort deponiert werden sollen. Der Vorteil der Aerosol-Druckgaspackungen besteht darin, daß bei ihrer Verwendung eine Wolke feinstzerteilter Partikel entsteht, die vom Patienten eingeatmet werden kann. In der Folge kommt es zu einem raschen Wirkungseintritt am Wirkort Lunge, was für die Therapie beispielsweise des Asthma bronchiale von entscheidender Wichtigkeit ist. Auf der anderen Seite kann bei der Prävention von Asthmaanfällen durch prophylaktisch wirksame Substanzen bei derartig lokaler Applikation direkt in die Lunge die Dosierung niedrig gehalten werden. Damit ist das Auftreten von unerwünschten Nebenwirkungen minimiert im Vergleich zur Applikation über den Magen/Darm-Trakt.

Aerosol-Druckgaspackungen haben daher eine weite Verbreitung in der Therapie von Atemwegserkrankungen gefunden. Sie sind einfach, sicher und preiswert. Mögliche Probleme bei der Koordination von Atemzug des Patienten und Auslösung eines Aerosolstoßes können entweder durch zwischen Aerosolpackung und Mund des Patienten eingeschobene Expansionskammern ("Spacer") oder durch spezielle Konstruktionen der Inhalatoren vermieden werden, bei denen der Einatemzug des Patienten den Aerosolstoß auslöst.
Neben der inhalativen Anwendung zur Prophylaxe von Asthma bronchiale und zur Therapie des akuten Asthmaanfalls kann die erfindungsgemäße Formulierung auch als Nasenspray und zur Anwendung als Mundspray (linguale und bukkale Applikation) gelangen.

Als Treibmittel für Dosieraerosole setzte man bisher FCKW ein (fluorierte chlorierte Kohlenwasserstoffe). Verwendbar als Treibmittel sind zum Beispiel folgende fluorierte chlorierte Kohlenwasserstoffe und Kohlenwasserstoffe: Pentan, n-Butan, iso-Butan, TG 11, TG 12, TG 21, TG 22, TG 23, TG 113, TG 114, TG 115, TG 142b und TG C 318.

Die Typenbezeichnung der fluorierten Chlorkohlenwaserstoffe leitet sich aus folgendem Schlüsselsystem ab:
- Zahl in Einerstelle =: Anzahl Fluoratome (F)
- Zahl in Zehnerstelle minus 1 =: Anzahl Wasserstoffatome (H)
- Zahl in Hunderterstelle plus 1 =: Anzahl Kohlenstoffatome (C)
- Zahl der noch freien Valenzen =: Anzahl Chloratome (Cl)

Seit Aufstellung der Ozontheorie (Abbau des stratosphärischen Ozons durch FCKW und andere chlorhaltige organische Verbindungen)sucht man als Treibmittel geeignete Flüssiggase, die weder brennbar, noch in der Lage sind, Ozon abzubauen und außerdem nicht gesundheitsschädlich sind.

Seit einiger Zeit arbeitet man mit nichtchlorierten Fluorkohlenwasserstoffen wie beispielsweise 1,1,1,2-Tetrafluorethan (TG 134a) oder 2H-Heptafluorpropan (TG 227).

Außer TG 134a und TG 227 wäre noch TG 152a (Difluorethan, CH₃CHF₂), TG 143a (Trifluorethan, CH₃CF₃) und TG 161 (Fluorethan, CH₃CH₂F) zu nennen.

Nachteilig an diesen Treibmitteln ist allerdings, daß zu ihrer Verwendung erforderliche Suspensionsstabilisatoren und Ventilschmiermittel nicht in ausreichendem Maße in ihnen löslich sind.
So erfordert die Verwendung von TG 134a etwa 25 % Ethanol, um das bisher in Aerosolsuspensionen verwendete Sorbitantrioleat (Span®85) in ausreichendem Maße zu lösen (s. EP 372 777 A2).
Beispielsweise können noch folgende Verbindungen verwendet werden: mehrwertige Alkohole wie zum Beispiel Glycerol, Ester wie zum Beispiel Ethylacetat, Ketone wie zum Beispiel Aceton und Kohlenwasserstoffe wie zum Beispiel Hexan und Heptan, Pentan und auch Isopropanol. Nachteilig bei einer derartig hohen Konzentration ist, daß es zu Auflösungserscheinungen für den in der Suspension befindlichen Wirkstoff kommen kann und damit die Gefahr des Teilchenwachstums besteht. Wachsen beim Lagern einer derartigen Suspension die Wirkstoffteilchen über eine Größe von 10 µm, so kann es einerseits zu Verstopfungen des Aerosolventils, auf der anderen Seite aber zu einer Wirkungsverringerung bis zu Unwirksamkeit des Aerosols kommen, da die Wirkstoffpartikel aufgrund ihrer Größe nicht mehr in der Lage sind, tiefere Lungenabschnitte zu erreichen.
Das Patent US 5,156,833 beschreibt eine Aerosolzusammensetzung aus einem Pulver, einem flüssigen Treibgas und einem nichtionischen Tensid. Die darin beanspruchten nichtionischen Tenside sind in dem eingesetzten Treibmittel unlöslich. Außerdem werden keine physiologischen Kriterien für die Tenside, wie physiologische Verträglichkeit und geschmackliche Akzeptanz gefordert, da die Zusammensetzung als Kosmetikum zur Anwendung auf der Haut gedacht ist.

Es besteht also ein dringender Bedarf nach Substanzen, die
- physiologisch verträglich sind
- technologisch geeignet sind, Aerosolsuspensionen von TG 134a oder TG 227 zu stabilisieren sowie die Funktion des Dosierventils zu verbessern
- in TG 134a oder TG 227 ohne oder unter Anwendung geringer Mengen anderer physiologisch verträglicher Lösungsvermittler löslich sind
- geschmacklich akzeptabel sind.
Es wurde nun überraschend gefunden, daß in der Klasse der Polyoxyethlen-glyceryl-fettsäureester geeignete Verbindungen zu finden sind, die die oben genannten Eigenschaften aufweisen und daher in der Lage sind, die Aufgabe zu lösen.

Es wurde gefunden, daß Polyoxyethlen(7)-Glycerylcocoat eine Substanz mit den eben erwähnten erforderlichen Eigenschaften darstellt. (Handelsname Cetiol HE, Hersteller: Henkel) PEG-7 Glyceryl Cocoate hat folgende Strukturformel: 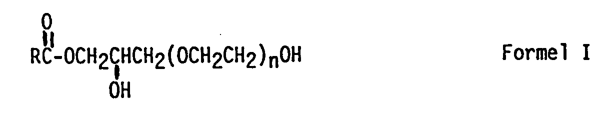 wobei gilt: n hat einen ungefähren Wert von 7. Weitere suspensionsstabilisierende Verbindungen, die die vorstehend erwähnten Eigenschaften aufweisen, sind:

Ein Polyoxyethlen(30)-Glycerylcocoat mit der Abkürzung PEG-30 Glyceryl Cocoat 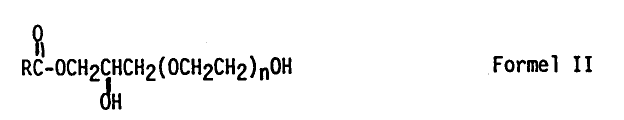 n hat einen Wert von ungefähr 30, der Handelsname der Verbindung lautet Varonic LI-63, der Hersteller Ashland Chemical Company. 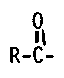 stellt in den Formeln I und II den Cocoat-Rest dar.

Ebenso kann die Verbindung mit dem Namen Polyoxyethylen(12)-Glyceryllaurat verwendet werden: 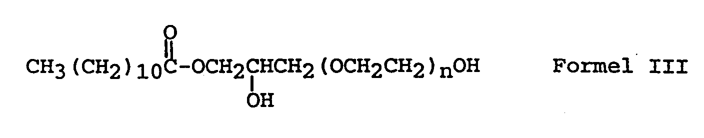 n hat einen durchschnittlichen Wert von 12, im Handel ist die Verbindung unter dem Namen Lamacit GML-12, Hersteller: Chemische Fabrik Grünau, erhältlich.

Weiter kann als Suspensionsstabilisator Polyoxyethylen(20)-Glyceryllaurat eingesetzt werden: 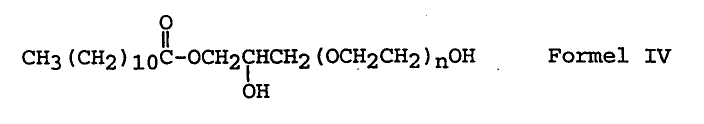 n hat einen durchschnittlichen Wert von 20. Im Handel ist es unter dem Namen Lamacit GML-20 von der Chemischen Fabrik Grünau oder unter dem Namen Tagat L2 von der Goldschmidt AG erhältlich. Tagat L2 hat einen HLB-Wert von 15,7 ± 1.

Weiterhin kann verwendet werden: Polyoxyethylen(30)-Glyceryllaurat 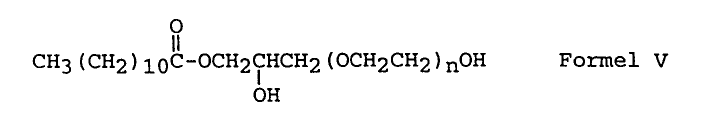 n hat einen durchschnittlichen Wert von 30. Im Handel ist es unter dem Handelsnamen Tagat L von der Firma Goldschmidt. Tagat L hat einen HLB-Wert von 17,0 ± 1.

Weiterhin eingesetzt werden kann Polyoxyethylen(25)-Glyceryloleat 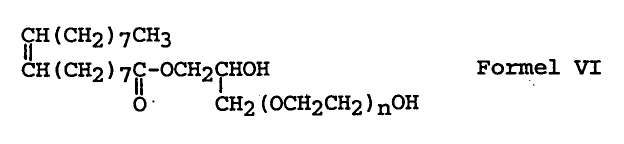 n hat einen durchschnittlichen Wert von 25. Im Handel ist die Verbindung unter dem Namen Lamacit GMO-25 von der Chemischen Fabrik Grünau.
Lamacit GMO-25 hat einen HLB-Wert von 16,2 ± 1.

Ebenso kann eingesetzt werden: Polyoxyethylen(15)-Glycerylricinoleate 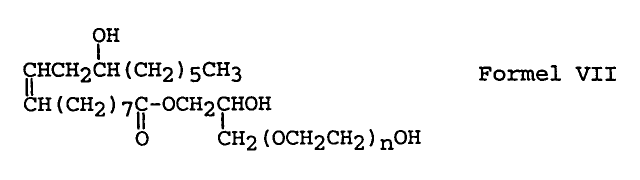 n hat einen durchschnittlichen Wert von 15. Im Handel ist diese Verbindung unter dem Namen Tagat R1,
Hersteller: Goldschmidt AG.
Tagat R1 hat einen HLB-Wert von 14 ± 1.

Weiter kann eingesetzt werden ein Polyoxyethylen(20)-Glycerylricinoleat gemäß der Formel VIII 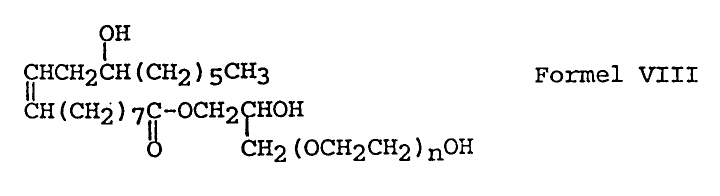 wobei n den Wert 20 hat. Im Handel ist die Verbindung unter dem Namen Lamacit ER, Hersteller: Chemische Fabrik, Grünau. Weiterhin kann eingesetzt werden ein Polyoxyethylen(5)-Glycerylstearat 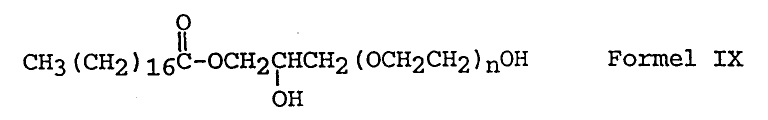 wobei der Wert n einen Wert von ungefähr 5 annehmen kann.

Diese Substanz ist im Handel unter dem Namen POEM-S-105 erhältlich.

Ebenso kann Polyoxyethlen(10)-Glycerylstearat 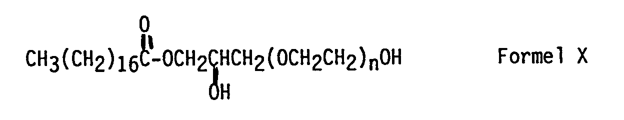 verwendet werden.
n hat einen durchschnittlichen Wert von 10.

Weiter kann Polyoxyethlen(20)-Glycerylstearat verwendet werden 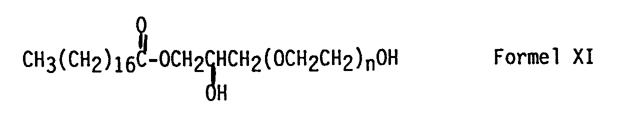 wobei n den Wert von 20 hat. Es ist im Handel unter dem Namen Cutina E-24 von Henkel und unter dem Namen Tagat S2 von der Goldschmidt AG. Tagat S2 hat einen HLB-Wert von 15,0 ± 1.

Ebenso kann Polyoxyethlen(30)-Glycerylstearat verwendet werden 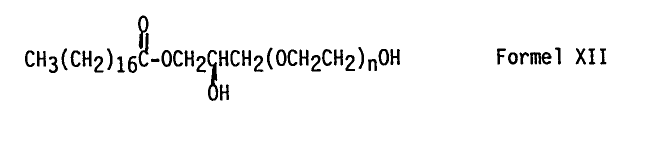 n kann einen Wert von ca. 30 annehmen.

Im Handel ist es unter dem Namen Tagat S von der Firma Goldschmidt AG.
Tagat S hat einen HLB-Wert von 16,4 ± 1.

Polyoxyethylen(120) -Glycerylstearat 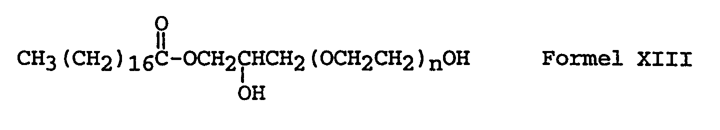 kann ebenfalls eingesetzt werden. n hat den durchschnittlichen Wert von 120. Im Handel ist es unter dem Namen Drewmulse 1128, Hersteller Drew Chemical Corporation.

Ebenso kann Polyoxyethylen(28)-Glyceryltallowate gemäß der Formel XIV verwendet werden 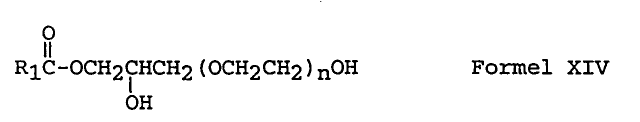 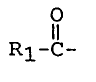 hat die Bedeutung von Tall-Säuren.

n hat den durchschnittlichen Wert von 28. Im Handel ist es unter dem Namen Varonic LI 2 von der Firma Ashland Chemical Company.

Weitere geeignete Verbindungen stellen folgende Polyoxyethlenglyceryl-fettsäureester dar:

Polyoxyethylen(30)-Glycerylmono-isostearat 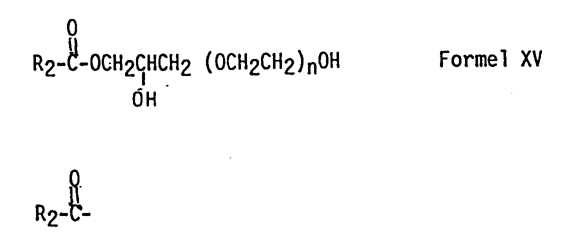 hat in Formel XV die Bedeutung des Isostearylrestes.

Die Ethylenoxykette ist ca. 30-40 Ethylenoxid-Einheiten (n= 30-40) lang. Die Substanz ist unter dem Namen Tagat I im Handel.

Es kann auch Polyoxyethlen-(20)-Glyceryl-monoisostearat 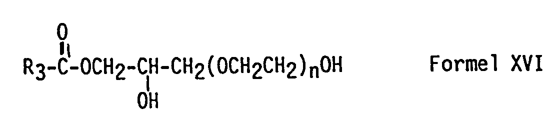 eingesetzt werden. 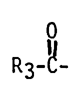 hat die Bedeutung des Isostearylrestes

Die Ethylenoxidkette ist ca. 20 Ethylenoxid-Einheiten (n ca. 20) lang. Die Substanz ist unter dem Namen Tagat I2 im Handel.

Weiter kann Polyoxyethlen-(40) Glyceryl-trihydroxystearat 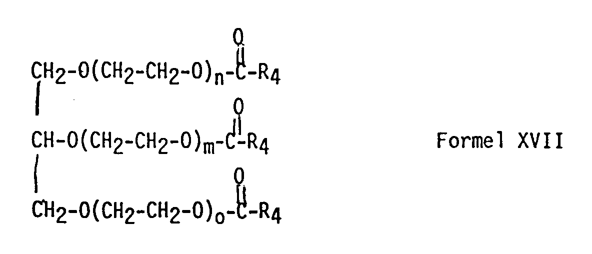 eingesetzt werden.

Die Summe aus m + n + o ist durchschnittlich 40 - 45

Unter 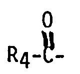 ist der 12-Hydroxystearylrest zu verstehen.
Die Substanz ist unter dem Namen Tagat R40 (Goldschmidt AG) oder Cremophore RH40 (BASF) im Handel.

Weiter kann Polyoxyethylen-(60)-Glyceryl-trihydroxystearat 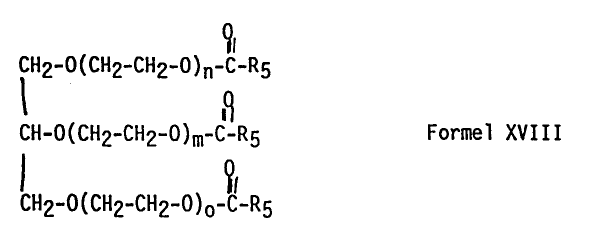 eingesetzt werden.
Die Summe aus n + m + o ist durchschnittlich 60

Unter 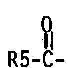 ist der 12-Hydroxystearylrest zu verstehen.
Die Substanz ist unter dem Namen Cremophor RH 60 (BASF-Wyandotte) oder Tagat R60 (Goldschmidt AG) im Handel.

Weiter kann Polyoxyethlen-(35)-Glyceryl-triricinoleat 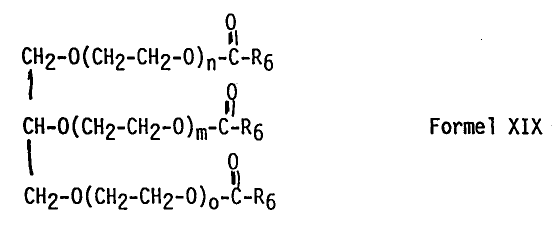 eingesetzt werden. Die Summe aus n + m + o ist durchschnittlich 35;
unter 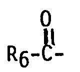 ist der Ricinolsäurerest zu verstehen.
Im Handel befindet sich die Substanz unter dem Namen Cremophor EL.

Polyoxyethlen-glyceryl-fettsäureester gemäß den Formeln I bis XIX sind
- physiologisch verträglich
- technologisch geeignet, Aerosolsuspension von TG 134a und/oder TG 227 zu stabilisieren sowie die Funktion des Dosierventils zu verbessern
- in TG 134a beziehungsweise TG 227 löslich unter gleichzeitiger Anwesenheit von weniger als 10 Vol%, vorzugsweise 1-2 Vol% Ethanol oder vergleichbarer Alkohole
- geschmacklich akzeptabel

Das Lösungsvermögen einer Mischung von TG 134a oder TG 227 mit 1-2 % Ethanol für die üblichen Wirksubstanzen ist derartig gering, daß es keine Rolle für ein mögliches Kristellwachstum der Wirkstoffe spielt. Die bisher in Handelspräparaten eingesetzten Suspensionsstabilisatoren hatten einen HLB-Wert von unter 5 (z.B. Span 85: HLB = 1.8) und liegen damit im Bereich der W/O-Emulgatoren. (Voigt, Lehrbuch der pharmazeutischen Technologie, Weinheim, 1987, S. 332).

Es ist daher überraschend, daß Substanzen wie Verbindungen gemäß den Formeln I - XIX mit einem HLB-Wert von > 10 für diesen Zweck geeignet sind.

Der HLB-Wert wird in Römpps Chemie-Lexikon, Stuttgart 1973, Band 3, s. 1478-1479 erläutert.

Die eingesetzte Menge an Verbindungen der Formel I bis XIX bezogen auf eine eingesetze Menge Wirkstoff beträgt beispielsweise zwischen 0,1 und 8000, insbesondere zwischen 5 und 4000, besonders bevorzugt zwischen 15 und 2500 Gewichts%.

Als Wirkstoffe können eingesetzt werden:

Analgetika, Antiallergika, Antibiotika, Anticholinergika, Antihistaminika, antiinflammatorisch wirkende Substanzen, Antitussiva, Bronchodilatatoren, Diuretika, Enzyme, Herz-Kreislauf wirksame Substanzen, Hormone, Proteine und Peptide. Beispiele für Analgetika sind Codein, Diamorphin, Dihydromorphin, Ergotamin, Fentanyl, Morphin; Beispiele für Antiallergika sind Cromo-glicinsäure, Nedocromil ; Beispiele für Antibiotika sind Cephalosporine, Fusafungin, Neomycin, Penicilline, Pentamidin, Streptomycin, Sulfonamide, Tetracycline; Beispiele für Anticholinergika sind Atropin, Atropinmethonitrat, Ipratropiumbromid, Oxitropiumbromid, Trospiumchlorid; Beispiele für Antihistaminika sind Azelastin, Flezelastin, Methapyrilen; Beispiele für antiinflammatorisch wirksame Substanzen sind Beclomethason, Budesonid, Dexamethason, Flunisolid, Fluticason, Tipredane, Triamcinolon; Beispiele für Antitussiva sind Narcotin, Noscapin; Beispiele für Bronchodilatatoren sind Bambuterol, Bitolterol, Carbuterol, Clenbuterol, Ephedrin, Epinephrin, Formoterol, Fenoterol, Hexoprenalin, Ibuterol, Isoprenalin, Isoproterenol, Metaproterenol, Orciprenalin, Phenylephrin, Phenylpropanolamin, Pirbuterol, Procaterol, Reproterol, Rimiterol, Salbutamol, Salmeterol, Sulfonterol , Terbutalin, Tolobuterol; Beispiele für Diuretika sind Amilorid, Furosemid; ein Beispiel für Enzyme ist Trypsin; Beispiele für HerzKreislauf wirksame Substanzen sind Diltiazem und Nitroglycerin; Beispiele für Hormone sind Cortison, Hydrocortison, Prednisolon; Beispiele für Proteine und Peptide sind Cyclosporine, Cetrorelix, Glucagon, Insulin. Weitere Wirkstoffe, die eingesetzt werden können, sind Adrenochrom, Colchicin, Heparin, Scopolamin.

Es können auch Kombinationen der vorstehend aufgeführten Substanzen eingesetzt werden.

Die beispielhaft angeführten Wirkstoffe können als freie Basen oder Säuren oder als pharmazeutisch verträgliche Salze eingesetzt werden. Als Gegenionen können beispielsweise physiologisch verträgliche Erdalkali- oder Alkalimetalle oder Amine sowie beispielsweise Azetat, Benzolsulfonat, Benzoat, Hydrogencarbonat, Hydrogentartrat, Bromid, Chlorid, Iodid, Karbonat, Citrat, Fumarat, Malat, Maleat, Gluconat, Lactat, Pamoat Hydroxynaphtoat und Sulphat eingesetzt werden. Es können auch Ester eingesetzt werden, zum Beispiel Acetat, Acetonid, Propionat, Dipropionat, Valerat.
Die Menge an Stoffen gemäß Formel I - XIX bezogen auf die Gesamtmischung, zusammengesetzt aus Wirkstoffen, Treibgas oder Teibgasgemischen, gegebenenfalls Hilfsstoffen, beträgt beispielsweise 0,01 Gew.% - 5 Gew.%, insbesondere
0,2 Gew.% - 2,5 Gew.% und besonders bevorzugt
0,75 Gew.% - 1,5 Gew.%.

Der Zusatz von Cosolventien ist möglich, beispielsweise aliphatische Alkohole mit 2 bis 6 C-Atomen oder deren Ester oder Ketone oder Polygole. Beispiele sind Ethanol, Isopropanol, Propylenglykol, Aceton, Essigsäurethylester, n-Propanol, vorzugsweise Ethanol und Isopropanol.

Die Menge an Ethanol oder Isopropanol, bezogen auf die Gesamtmischung, beträgt zwischen 0 Gew.% bis 10 Gew.%, insbesondere 0,1 Gew.% bis 2 Gew.% und besonders bevorzugt 0,2 Gew.% bis 1 Gew.%.

Der Zusatz weiterer grenzflächenaktiver Substanzen, wie beispielsweise in EP 0 372 777 aufgeführt, ist selbstverständlich möglich.

Die Suspendierung der Wirkstoffe kann entweder erfolgen bei normalem Luftdruck, wobei das Suspensionsmedium auf niedrige Temperaturen abgekühlt werden muß (zum Beispiel - 35°C bis - 55°C) oder innerhalb eines Druckgefaßes, wobei bei Normaltemperaturen (Raumtemperatur 15° bis 25°C) gearbeitet werden kann.

Die Suspension wird homogenisiert und anschließend in Druckdosen abgefüllt, die mit einem Dosierventil verschlossen sind oder anschließend verschlossen werden.

### Beispiel 1

1000 g 2H-Heptafluorpropan (= Treibmittel 227) werden auf eine Temperatur von etwa -55°C abgekühlt und unter Rühren mit einer Lösung aus aus 11,7 g Polyoxyethylen(20)-glyceryl-Monolaurat (Handelsname: Tagat L2, Goldschmidt AG) in 11,7 g absolutem Ethanol versetzt. Anschließend werden 16,8 g mikronisierte Cromoglicinsäure, Dinatriumsalz und 8,4 g mikronisiertes Reproterolhydrochlorid sowie 0,9 g mikronisiertes Saccharin-Natrium und 6,75 g Pfefferminzöl zugesetzt und die entstandene Suspension intensiv homogenisiert. Unter weiterem Rühren und Kühlen wird die Suspension mit gekühltem Treibmittel 227 auf 1170,0 g aufgefüllt und sodann in Metalldosen abgefüllt, die mit Dosierventilen verschlossen werden, welche pro Hub 50 µl der Suspension freisetzen. Pro Hub werden damit 1 mg Cromoglicinsäure, Dinatriumsalz und 0,5 mg Reproterolhydrochlorid freigesetzt.

### Beispiel 2

900 g 2H-Heptafluorpropan (=Treibmittel 227) werden auf eine Temperatur von etwa -55°C abgekühlt und unter Rühren mit einer Lösung aus 11,7 g Polyoxyethylen (30)-Glyceryl Stearat (Handelsname: Tagat®S, Goldschmidt AG) in 117 g absolutem Ethanol versetzt. Anschließend werden 8,4 g mikronisiertes Reproterolhydrochlorid sowie 0,9 g mikronisiertes Saccharin-Natrium und 6,75 g Pfefferminzöl zugesetzt und die entstandene Suspension intensiv homogenisiert.

Unter weiterem Rühren und Kühlen wird die Suspension mit gekühltem Treibmittel 227 auf 1170,0 g aufgefüllt und sodann in Metalldosen abgefüllt, die mit Dosierventilen geschlossen werden, welche pro Hub 50 µl der Suspension freisetzen. Pro Hub werden damit 0,5 mg Reproterolhydrochlorid freigesetzt.

### Beispiel 3

Es wird gearbeitet wie in Beispiel 1, jedoch wird statt 16,8 g mikronisiertem Cromoglicinsäure, Dinatriumsalz, 8,4 g mikronisiertem Reproterolhydrochlorid, 0,9 g mikronisiertem Saccharin-Natrium und 6,75 g Pefferminzöl 4,2 g mikronisiertes Budesonid eingesetzt.
Ein Hub enthält 0,25 mg Budesonid.

## Patentansprüche

1. Aerosol-Druckgaspackungen zur inhalativen Verabreichung von biologisch aktiven Substanzen, dadurch gekennzeichnet, daß Polyoxyethylen(7)-Glycerylcocoat gemäß Formel I 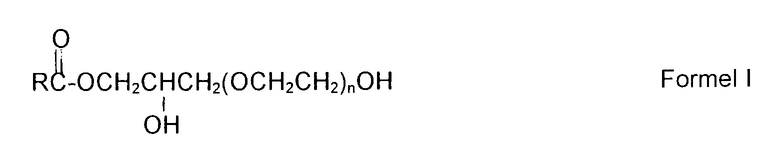 wobei für n etwa 7 gilt und 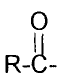 für den Cocoat-Rest steht, als Suspensionsstabilisator und/oder Ventilschmiermittel enthalten ist.

2. Aerosol-Druckgaspackungen zur inhalativen Verabreichung von biologisch aktiven Substanzen, dadurch gekennzeichnet, daß Polyoxyethylen(30)-Glycerylcocoat gemäß Formel II 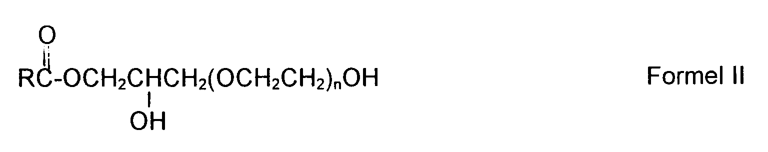 wobei für n etwa 30 gilt und 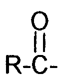 für den Cocoat-Rest steht, als Suspensionsstabilisator und/oder Ventilschmiermittel enthalten ist.

3. Aerosol-Druckgaspackungen zur inhalativen Verabreichung von biologisch aktiven Substanzen, dadurch gekennzeichnet, daß Polyoxyethylen(12)-Glyceryllaurat gemäß Formel II 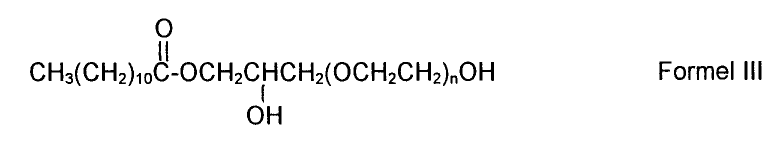 wobei für n etwa 12 gilt, als Suspensionsstabilisator und/oder Ventilschmiermittel enthalten ist.

4. Aerosol-Druckgaspackungen zur inhalativen Verabreichung von biologisch aktiven Substanzen, dadurch gekennzeichnet, daß Polyoxyethylen(20)-Glyceryllaurat gemäß Formel IV 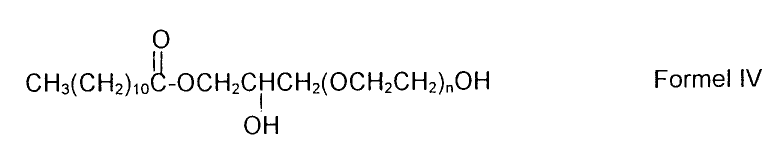 wobei für n etwa 20 gilt, als Suspensionsstabilisator und/oder Ventilschmiermittel enthalten ist.

5. Aerosol-Druckgaspackungen zur inhalativen Verabreichung von biologisch aktiven Substanzen, dadurch gekennzeichnet, daß Polyoxyethylen(30)-Glyceryllaurat gemäß Formel V 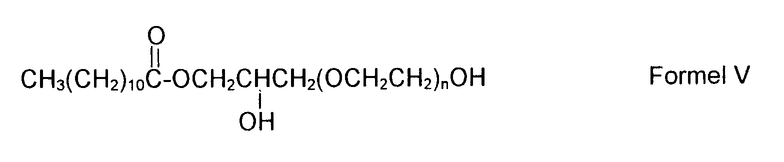 wobei für n etwa 30 gilt, als Suspensionsstabilisator und/oder Ventilschmiermittel enthalten ist.

6. Aerosol-Druckgaspackungen zur inhalativen Verabreichung von biologisch aktiven Substanzen, dadurch gekennzeichnet, daß Polyoxyethylen(25)-Glyceryloleat gemäß Formel VI 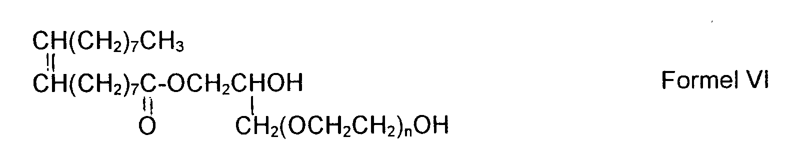 wobei für n etwa 25 gilt, als Suspensionsstabilisator und/oder Ventilschmiermittel enthalten ist.

7. Aerosol-Druckgaspackungen zur inhalativen Verabreichung von biologisch aktiven Substanzen, dadurch gekennzeichnet, daß Polyoxyethylen(15)-Glycerylricinoleat gemäß Formel VII 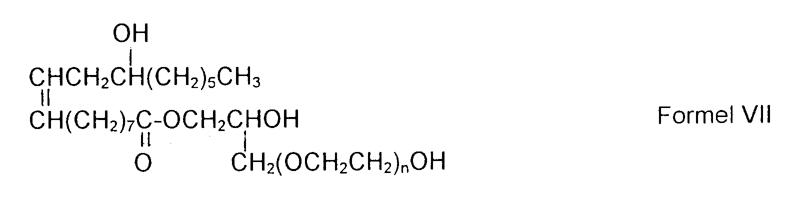 wobei für n etwa 15 gilt, als Suspensionsstabilisator und/oder Ventilschmiermittel enthalten ist.

8. Aerosol-Druckgaspackungen zur inhalativen Verabreichung von biologisch aktiven Substanzen, dadurch gekennzeichnet, daß Polyoxyethylen(20)-Glycerylricinoleat gemäß Formel VIII 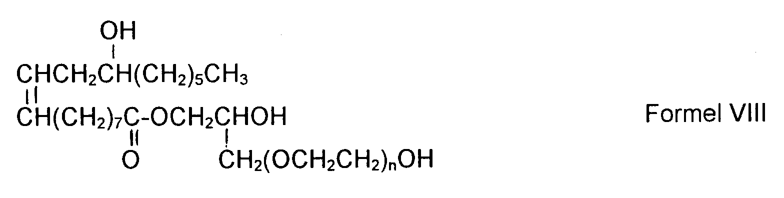 wobei für n etwa 20 gilt, als Suspensionsstabilisator und/oder Ventilschmiermittel enthalten ist.

9. Aerosol-Druckgaspackungen zur inhalativen Verabreichung von biologisch aktiven Substanzen, dadurch gekennzeichnet, daß Polyoxyethylen(5)-Glycerylstearat gemäß Formel IX 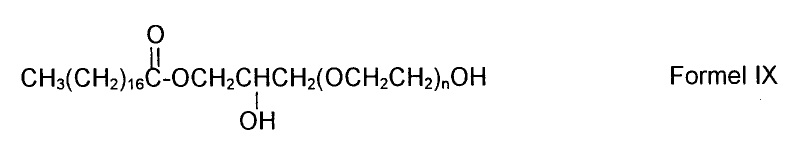 wobei für n etwa 5 gilt, als Suspensionsstabilisator und/oder Ventilschmiermittel enthalten ist.

10. Aerosol-Druckgaspackungen zur inhalativen Verabreichung von biologisch aktiven Substanzen, dadurch gekennzeichnet, daß Polyoxyethylen(10)-Glycerylstearat gemäß Formel X 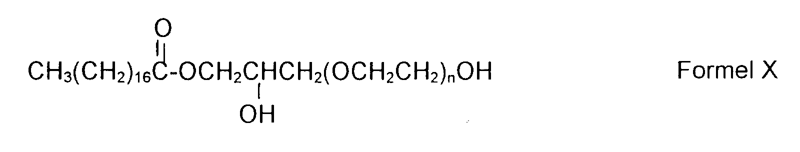 wobei für n etwa 10 gilt, als Suspensionsstabilisator und/oder Ventilschmiermittel enthalten ist.

11. Aerosol-Druckgaspackungen zur inhalativen Verabreichung von biologisch aktiven Substanzen, dadurch gekennzeichnet, daß Polyoxyethylen(20)-Glycerylstearat gemäß Formel XI 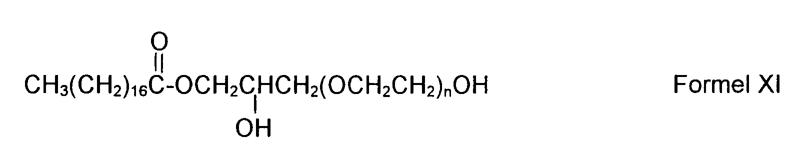 wobei für n = 20 gilt, als Suspensionsstabilisator und/oder Ventilschmiermittel enthalten ist.

12. Aerosol-Druckgaspackungen zur inhalativen Verabreichung von biologisch aktiven Substanzen, dadurch gekennzeichnet, daß Polyoxyethylen(30)-Glycerylstearat gemäß Formel XII 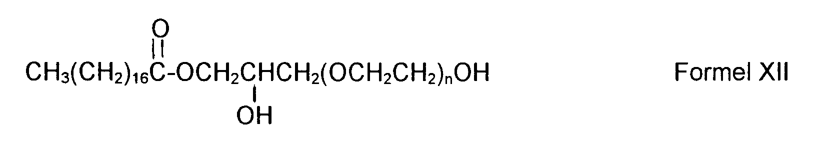 wobei für n etwa 30 gilt, als Suspensionsstabilisator und/oder Ventilschmiermittel enthalten ist.

13. Aerosol-Druckgaspackungen zur inhalativen Verabreichung von biologisch aktiven Substanzen, dadurch gekennzeichnet, daß Polyoxyethylen(120)-Glycerylstearat gemäß Formel XIII 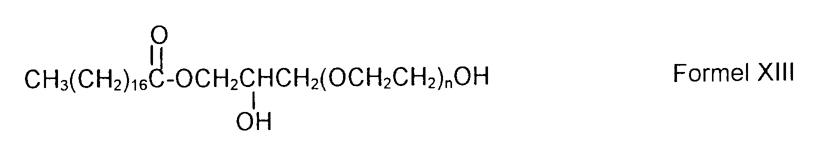 wobei für n etwa 120 gilt, als Suspensionsstabilisator und/oder Ventilschmiermittel enthalten ist.

14. Aerosol-Druckgaspackungen zur inhalativen Verabreichung von biologisch aktiven Substanzen, dadurch gekennzeichnet, daß Polyoxyethylen(28)-Glyceryl-tallowate gemäß Formel XIV 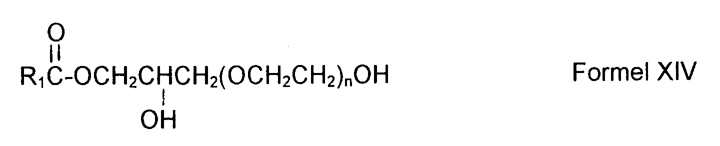 wobei für n etwa 28 gilt und 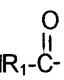 für Tall-Säuren steht, als Suspensionsstabilisator und/oder Ventilschmiermittel enthalten ist.

15. Aerosol-Druckgaspackungen zur inhalativen Verabreichung von biologisch aktiven Substanzen, dadurch gekennzeichnet, daß Polyoxyethylen(30)-Glycerylmonoisostearat gemäß Formel XV 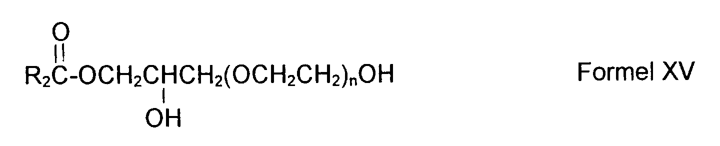 wobei für n etwa 30-40 gilt und 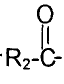 für den Isostearylrest steht, als Suspensionsstabilisator und/oder Ventilschmiermittel enthalten ist.

16. Aerosol-Druckgaspackungen zur inhalativen Verabreichung von biologisch aktiven Substanzen, dadurch gekennzeichnet, daß Polyoxyethylen(20)-Glyceryl-monoisostearat gemäß Formel XVI 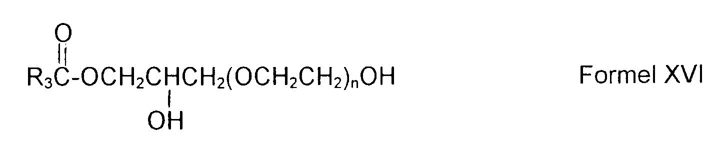 wobei für n etwa 20 gilt und 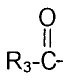 für den Isostearylrest steht, als Suspensionsstabilisator und/oder Ventilschmiermittel enthalten ist.

17. Aerosol-Druckgaspackungen zur inhalativen Verabreichung von biologisch aktiven Substanzen, dadurch gekennzeichnet, daß Polyoxyethylen(40)-Glyceryl-trihydroxystearat gemäß Formel XVII 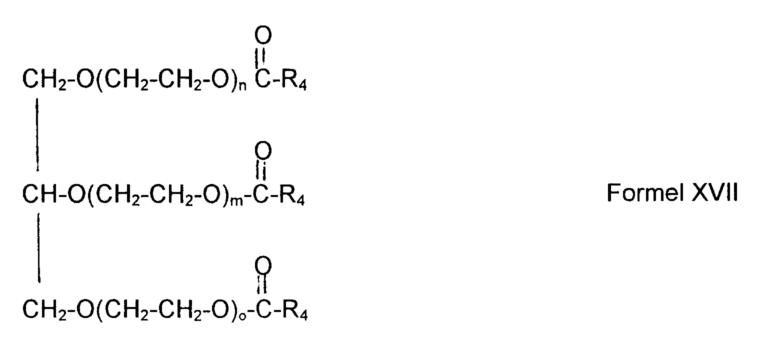 wobei für m+n+o etwa 40-45 gilt und 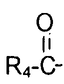 für den 12-Hydroxystearylrest steht, als Suspensionsstabilisator und/oder Ventilschmiermittel enthalten ist.

18. Aerosol-Druckgaspackungen zur inhalativen Verabreichung von biologisch aktiven Substanzen, dadurch gekennzeichnet, daß Polyoxyethylen(60)-Glyceryl-trihydroxystearat gemäß Formel XVIII 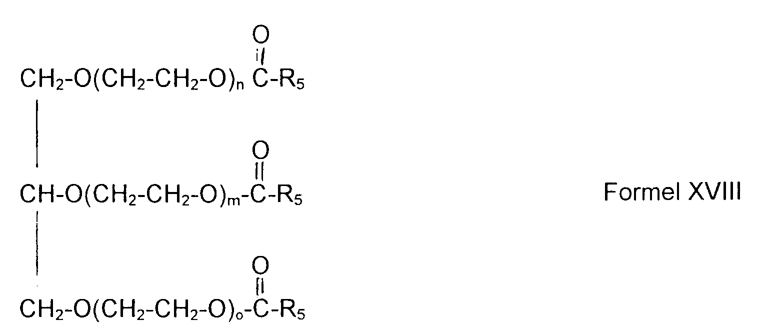 wobei für m+n+o etwa 60 gilt und 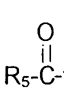 für den Hydroxystearylrest steht, als Suspensionsstabilisator und/oder Ventilschmiermittel enthalten ist.

19. Aerosol-Druckgaspackungen zur inhalativen Verabreichung von biologisch aktiven Substanzen, dadurch gekennzeichnet, daß Polyoxyethylen(35)-Glyceryl-triricinoleat gemäß Formel XIX 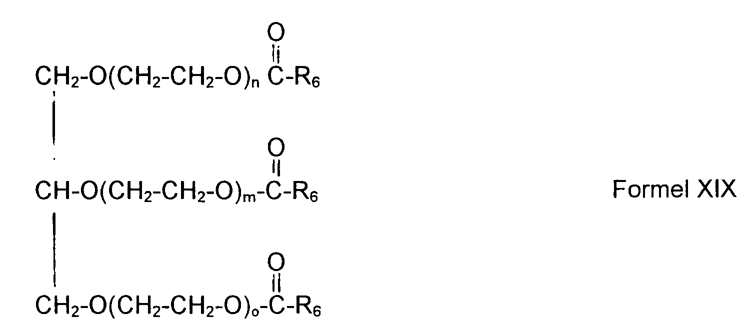 wobei für m+n+o etwa 35 gilt und 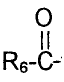 für den Ricinolsäurerest steht, als Suspensionsstabilisator und/oder Ventilschmiermittel enthalten ist.

20. Aerosol-Druckgaspackungen gemäß Anspruch 1 - 19, dadurch gekennzeichnet, daß der Anteil an Substanzen gemäß Formel I - XIX, bezogen auf das Gesamtgewicht der Mischung, zwischen 0,01 und 5 Gewichts % liegt.

21. Aerosol-Druckgaspackungen gemäß Anspruch 1 - 19, dadurch gekennzeichnet, daß der Anteil an Verbindungen der Formel I - XIX, bezogen auf das Gesamtgewicht der Mischung, zwischen 0,2 und 2,5 Gewichts % liegt.

22. Aerosol-Druckgaspackungen gemäß Anspruch 1 - 19, dadurch gekennzeichnet, daß der Anteil an Verbindungen der Formel I -XIX, bezogen auf das Gesamtgewicht der Mischung, zwischen 0,75 und 1,5 Gewichts % liegt.

23. Aerosol-Druckgaspackungen gemäß Anspruch 1 - 22 dadurch gekennzeichnet, daß als Treibmittel TG 227 und/oder TG 134a enthalten ist.

24. Verfahren zur Herstellung von zur inhalativen Verabreichung geeigneten Aerosol-Druckgaspackungen, dadurch gekennzeichnet, daß man einen Suspensionsstabilisator und/oder Ventilschmiermittel gemäß den Ansprüchen 1 - 19 verwendet.

## Claims

1. Aerosol pressurized gas packagings for the administration of biologically active substances by inhalation, characterized in that polyoxyethylene(7) glyceryl cocoate of formula I: 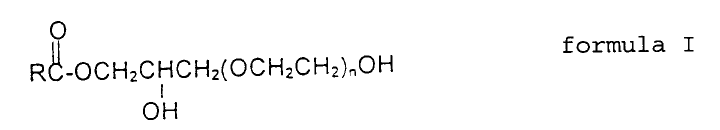 in which n is about 7 and 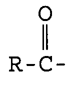 is the cocoate radical, is present as a suspension stabilizer and/or valve lubricant.

2. Aerosol pressurized gas packagings for the administration of biologically active substances by inhalation, characterized in that polyoxyethylene(30) glyceryl cocoate of formula II: 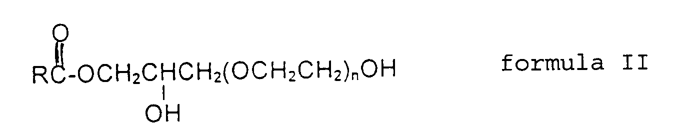 in which n is about 30 and 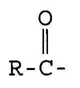 is the cocoate radical, is present as a suspension stabilizer and/or valve lubricant.

3. Aerosol pressurized gas packagings for the administration of biologically active substances by inhalation, characterized in that polyoxyethylene(12) glyceryl laurate of formula II: 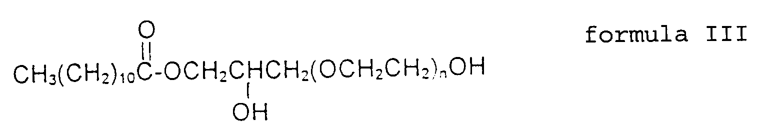 in which n is about 12, is present as a suspension stabilizer and/or valve lubricant.

4. Aerosol pressurized gas packagings for the administration of biologically active substances by inhalation, characterized in that polyoxyethylene(20) glyceryl laurate of formula IV: 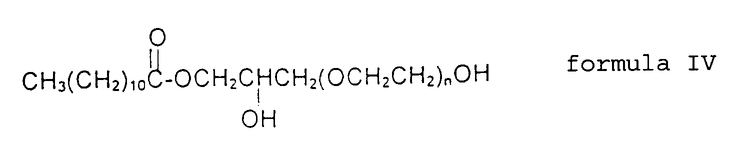 in which n is about 20, is present as a suspension stabilizer and/or valve lubricant.

5. Aerosol pressurized gas packagings for the administration of biologically active substances by inhalation, characterized in that polyoxyethylene(30) glyceryl laurate of formula V: 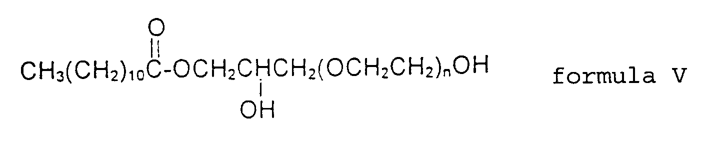 in which n is about 30, is present as a suspension stabilizer and/or valve lubricant.

6. Aerosol pressurized gas packagings for the administration of biologically active substances by inhalation, characterized in that polyoxyethylene(25) glyceryl oleate of formula VI: 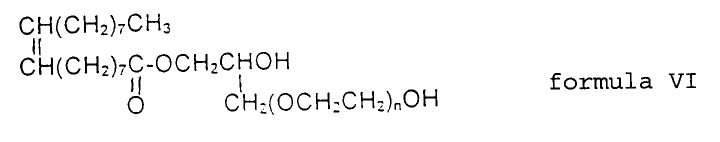 in which n is about 25, is present as a suspension stabilizer and/or valve lubricant.

7. Aerosol pressurized gas packagings for the administration of biologically active substances by inhalation, characterized in that polyoxyethylene(15) glyceryl ricinoleate of formula VII: 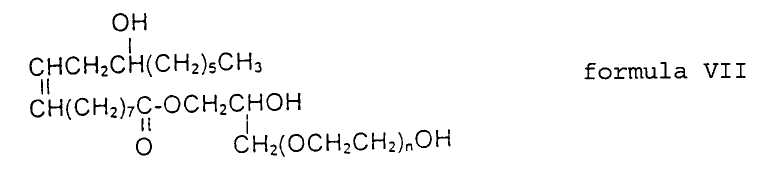 in which n is about 15, is present as a suspension stabilizer and/or valve lubricant.

8. Aerosol pressurized gas packagings for the administration of biologically active substances by inhalation, characterized in that polyoxyethylene(20) glyceryl ricinoleate of formula VIII: 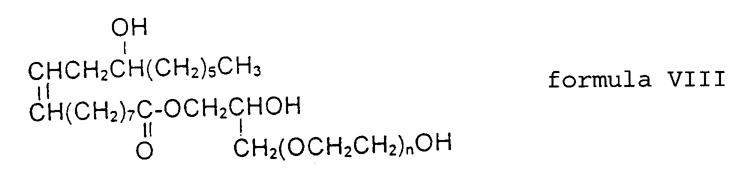 in which n is about 20, is present as a suspension stabilizer and/or valve lubricant.

9. Aerosol pressurized gas packagings for the administration of biologically active substances by inhalation, characterized in that polyoxyethylene(5) glyceryl stearate of formula IX: 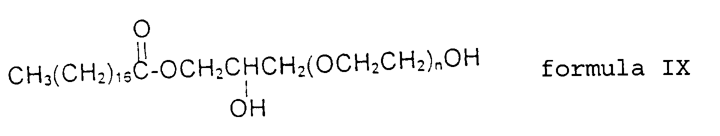 in which n is about 5, is present as a suspension stabilizer and/or valve lubricant.

10. Aerosol pressurized gas packagings for the administration of biologically active substances by inhalation, characterized in that polyoxyethylene(10) glyceryl stearate of formula X: 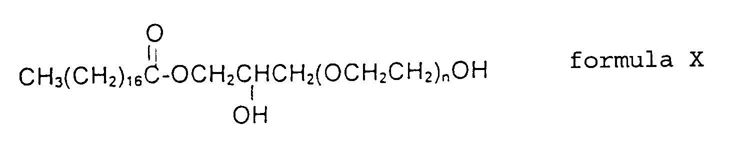 in which n is about 10, is present as a suspension stabilizer and/or valve lubricant.

11. Aerosol pressurized gas packagings for the administration of biologically active substances by inhalation, characterized in that polyoxyethylene(20) glyceryl stearate of formula XI: 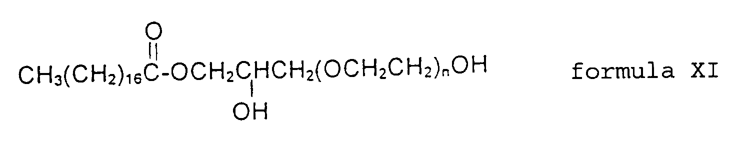 in which n = 20, is present as a suspension stabilizer and/or valve lubricant.

12. Aerosol pressurized gas packagings for the administration of biologically active substances by inhalation, characterized in that polyoxyethylene(30) glyceryl stearate of formula XII: 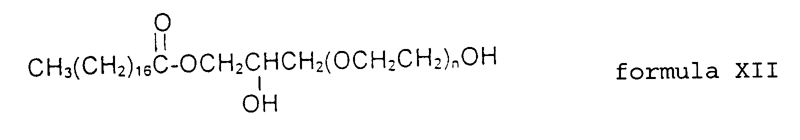 in which n is about 30, is present as a suspension stabilizer and/or valve lubricant.

13. Aerosol pressurized gas packagings for the administration of biologically active substances by inhalation, characterized in that polyoxyethylene(120) glyceryl stearate of formula XIII: 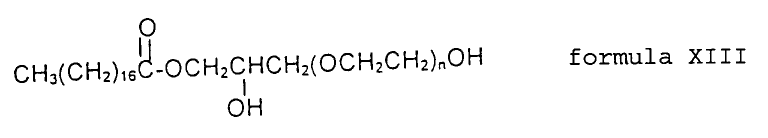 in which n is about 120, is present as a suspension stabilizer and/or valve lubricant.

14. Aerosol pressurized gas packagings for the administration of biologically active substances by inhalation, characterized in that polyoxyethylene(28) glyceryl tallowate of formula XIV: 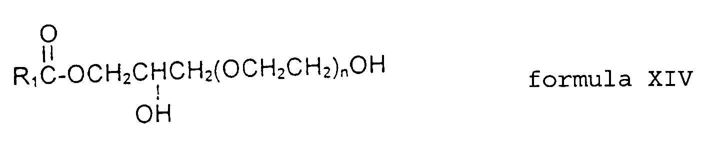 in which n is about 28 and 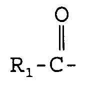 denotes tallow acids, is present as a suspension stabilizer and/or valve lubricant.

15. Aerosol pressurized gas packagings for the administration of biologically active substances by inhalation, characterized in that polyoxyethylene(30) glyceryl monoisostearate of formula XV: 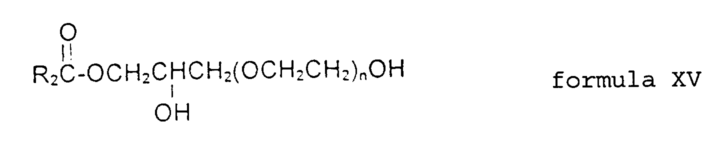 in which n is about 30 - 40 and 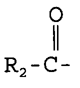 is the isostearyl radical, is present as a suspension stabilizer and/or valve lubricant.

16. Aerosol pressurized gas packagings for the administration of biologically active substances by inhalation, characterized in that polyoxyethylene(20) glyceryl monoisostearate of formula XVI: 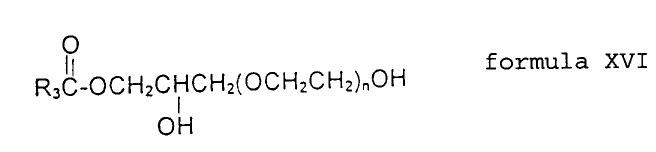 in which n is about 20 and 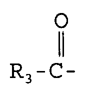 is the isostearyl radical, is present as a suspension stabilizer and/or valve lubricant.

17. Aerosol pressurized gas packagings for the administration of biologically active substances by inhalation, characterized in that polyoxyethylene(40) glyceryl trihydroxystearate of formula XVII: 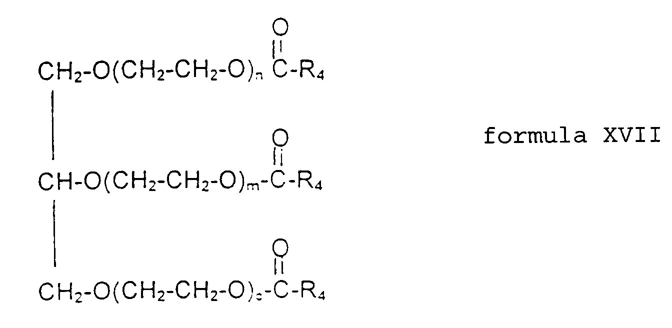 in which m + n + o is about 40 - 45 and 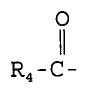 is the 12-hydroxystearyl radical, is present as a suspension stabilizer and/or valve lubricant.

18. Aerosol pressurized gas packagings for the administration of biologically active substances by inhalation, characterized in that polyoxyethylene(60) glyceryl trihydroxystearate of formula XVIII: 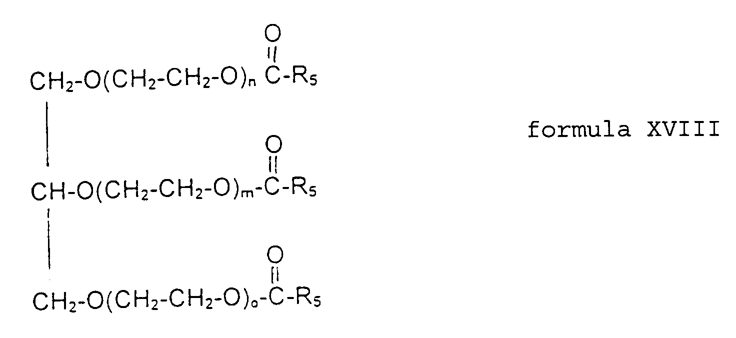 in which m + n + o is about 60 and 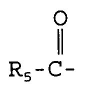 is the hydroxystearyl radical, is present as a suspension stabilizer and/or valve lubricant.

19. Aerosol pressurized gas packagings for the administration of biologically active substances by inhalation, characterized in that polyoxyethylene(35) glyceryl triricinoleate of formula XIX: 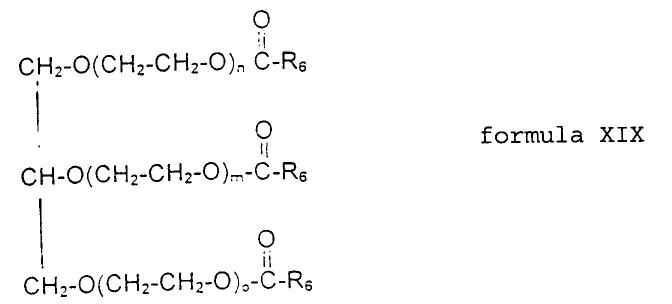 in which m + n + o is about 35 and 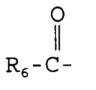 is the ricinoleic acid radical, is present as a suspension stabilizer and/or valve lubricant.

20. Aerosol pressurized gas packagings according to Claims 1 - 19, characterized in that the proportion of substances of formulae I - XIX, based on the total weight of the mixture, is between 0.01 and 5 wt.%.

21. Aerosol pressurized gas packagings according to Claims 1 - 19, characterized in that the proportion of compounds of formulae I - XIX, based on the total weight of the mixture, is between 0.2 and 2.5 wt.%.

22. Aerosol pressurized gas packagings according to Claims 1 - 19, characterized in that the proportion of compounds of formulae I - XIX, based on the total weight of the mixture, is between 0.75 and 1.5 wt.%.

23. Aerosol pressurized gas packagings according to Claims 1 - 22, characterized in that TG 227 and/or TG 134a is present as the propellant.

24. Process for the preparation of aerosol pressurized gas packagings suitable for administration by inhalation, characterized in that a suspension stabilizer and/or valve lubricant according to Claims 1 - 19 are used.

## Revendications

1. Conditionnements pour gaz comprimés en vue de l'administration par inhalation de substances biologiquement actives,
caractérisés en ce que
du cocoate de polyoxyéthylène (7) glycéryle conformément à la formule I : 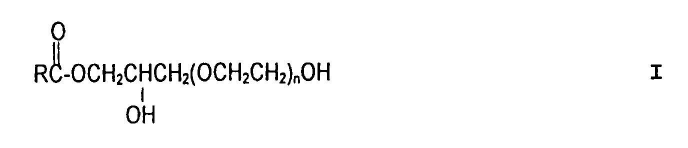 dans laquelle n vaut environ 7 et 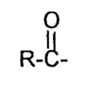 représente le radical cocoate,
est contenu en tant qu'agent stabilisant pour suspension et/ou lubrifiant pour valve.

2. Conditionnements pour gaz comprimés en aérosol en vue de l'administration par inhalation de substances biologiquement actives,
caractérisés en ce que
du polyoxyéthylène (30) glycéryle cocoate conformément à la formule II : 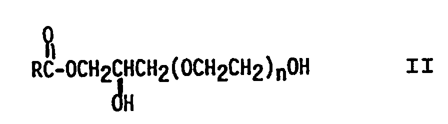 dans laquelle n vaut environ 30 et 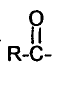 représente le radical cocoate,
est contenu en tant qu'agent stabilisant pour suspension et/ou comme lubrifiant pour valve.

3. Conditionnements pour gaz comprimés en aérosol en vue de l'administration par inhalation de substances biologiquement actives,
caractérisés en ce que
du polyoxyéthylène (12) glycéryle laurate conformément à la formule III : 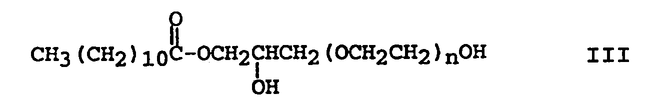 dans laquelle n vaut environ 12,
est contenu en tant qu'agent stabilisant pour suspension et/ou en tant que lubrifiant pour valve.

4. Conditionnement pour gaz comprimés en aérosol en vue de l'administration par inhalation de substances biologiquement actives,
caractérisés en ce que
du polyoxyéthylène (20) glycéryle laurate conformément à la formule IV : 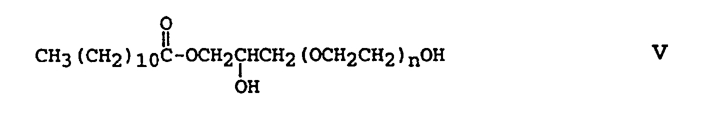 dans laquelle n vaut environ 20,
est contenu en tant qu'agent stabilisant pour suspension et/ou en tant que lubrifiant pour valve.

5. Conditionnements pour gaz comprimés en aérosol en vue de l'administration par inhalation de substances biologiquement actives,
caractérisés en ce que
du polyoxyéthylène (30) glycéryle laurate conformément à la formule V : 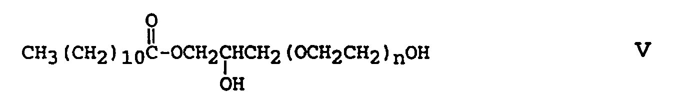 dans laquelle n vaut environ 30,
est contenu en tant qu'agent stabilisant pour suspension et/ou en tant que lubrifiant pour valve.

6. Conditionnements pour gaz comprimés en aérosol en vue de l'administration par inhalation de substances biologiquement actives,
caractérisés en ce que
du polyoxyéthylène (25) glycéryle oléate conformément à la formule VI : 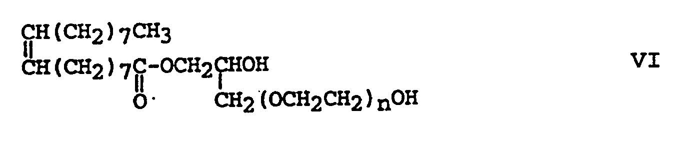 dans laquelle n vaut environ 25,
est contenu en tant qu'agent stabilisant pour suspension et/ou en tant que lubrifiant pour valve.

7. Conditionnements pour gaz comprimés en aérosol en vue de l'administration par inhalation de substances biologiquement actives,
caractérisés en ce que
du polyoxyéthylène (15) glycéryle ricinoléate conformément à la formule VII : 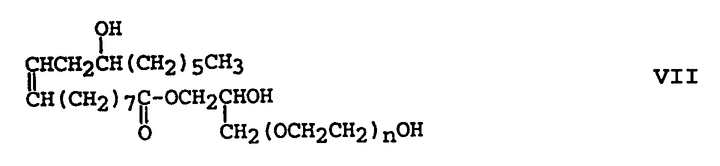 dans laquelle n vaut environ 15,
est contenu en tant qu'agent stabilisant pour suspension et/ou en tant que lubrifiant pour valve.

8. Conditionnements pour gaz comprimés en aérosol en vue de l'administration par inhalation de substances biologiquement actives,
caractérisés en ce que
du polyoxyéthylène (20) glycéryle ricinoléate conformément à la formule VIII : 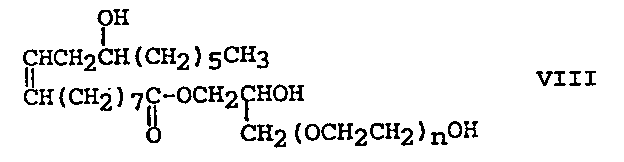 dans laquelle n vaut environ 20,
est contenu en tant qu'agent stabilisant pour suspension et/ou en tant que lubrifiant pour valve.

9. Conditionnements pour gaz comprimés en aérosol en vue de l'administration par inhalation de substances biologiquement actives,
caractérisés en ce que
du polyoxyéthylène (5) glycéryle stéarate conformément à la formule IX : 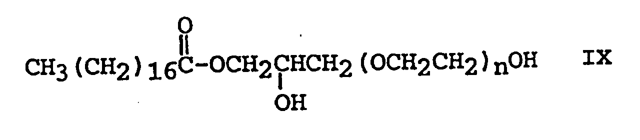 dans laquelle n vaut environ 5,
est contenu en tant qu'agent stabilisant pour suspension et/ou en tant que lubrifiant pour valve.

10. Conditionnements pour gaz comprimés en aérosol en vue de l'administration par inhalation de substances biologiquement actives,
caractérisés en ce que
du polyoxyéthylène (10) glycéryle stéarate conformément à la formule X : 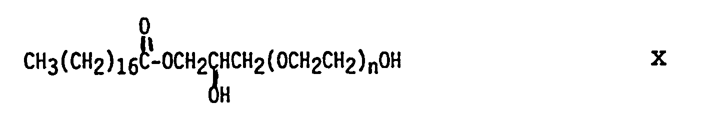 dans laquelle n vaut environ 10,
est contenu en tant qu'agent stabilisant pour suspension et/ou en tant que lubrifiant pour valve.

11. Conditionnements pour gaz comprimés en aérosol en vue de l'administration par inhalation de substances biologiquement actives,
caractérisés en ce que
du polyoxyéthylène (20) glycéryle stéarate conformément à la formule XI : 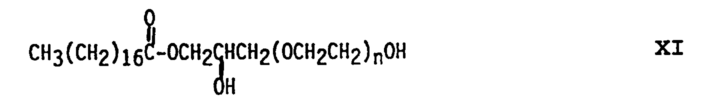 dans laquelle n vaut 20,
est contenu en tant qu'agent stabilisant pour suspension et/ou en tant que lubrifiant pour valve.

12. Conditionnements pour gaz comprimés en aérosol en vue de l'administration par inhalation de substances biologiquement actives,
caractérisés en ce que
du polyoxyéthylène (30) glycéryle stéarate conformément à la formule XII : 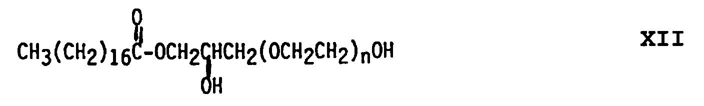 dans laquelle n vaut environ 30,
est contenu en tant qu'agent stabilisant pour suspension et/ou en tant que lubrifiant pour valve.

13. Conditionnements pour gaz comprimés en aérosol en vue de l'administration par inhalation de substances biologiquement actives,
caractérisés en ce que
du polyoxyéthylène (120) glycéryle stéarate conformément à la formule XIII : 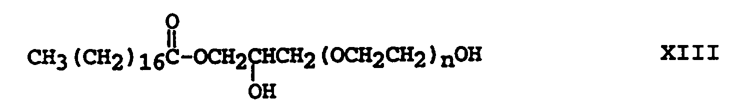 dans laquelle n vaut environ 120,
est contenu en tant qu'agent stabilisant pour suspension et/ou en tant que lubrifiant pour valve.

14. Conditionnements pour gaz comprimés en aérosol en vue de l'administration par inhalation de substances biologiquement actives,
caractérisés en ce que
du polyoxyéthylène (28) glycéryle tallowate conformément à la formule XIV : 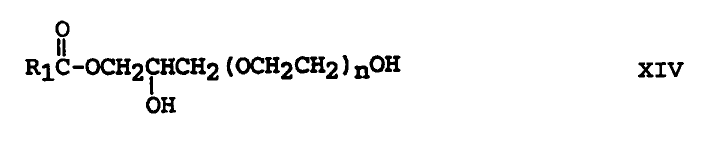 dans laquelle n vaut environ 28, et 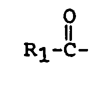 a la signification d'acides talliques,
est contenu en tant qu'agent stabilisant pour suspension et/ou en tant que lubrifiant pour valve.

15. Conditionnements pour gaz comprimés en aérosol en vue de l'administration par inhalation de substances biologiquement actives,
caractérisés en ce que
du polyoxyéthylène (30) glycéryle monoisostéarate conformément à la formule XV : 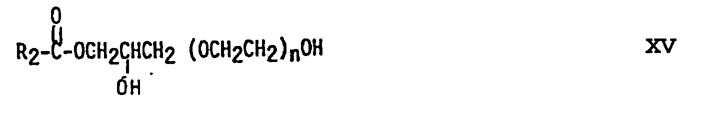 dans laquelle n vaut environ 30-40, et 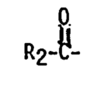 représente le radical isostéaryle,
est contenu en tant qu'agent stabilisant pour suspensions et/ou en tant que lubrifiant pour valve.

16. Conditionnements pour gaz comprimés en aérosol en vue de l'administration par inhalation de substances biologiquement actives,
caractérisés en ce que
du polyoxyéthylène (20) glycéryle monoisostéarate conformément à la formule XVI : 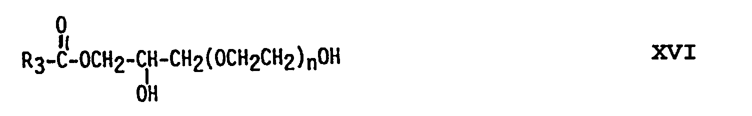 dans laquelle n vaut environ 20 et 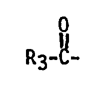 représente un radical isostéaryle,
est contenu en tant qu'agent stabilisant pour suspensions et/ou en tant que lubrifiant pour valve.

17. Conditionnements pour gaz comprimés en aérosol en vue de l'administration par inhalation de substances biologiquement actives,
caractérisés en ce que
du polyoxyéthylène (40) glycéryle trihydroxystéarate conformément à la formule XVII : 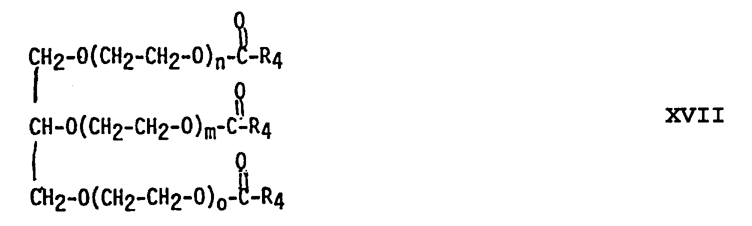 dans laquelle m+n+o vaut environ 40-45, et 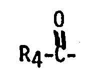 représente un reste 12-hydroxystéaryle,
est contenu en tant qu'agent stabilisant pour suspension et/ou en tant que lubrifiant pour valve.

18. Conditionnements pour gaz comprimés en aérosol en vue de l'administration par inhalation de substances biologiquement actives,
caractérisés en ce que
du polyoxyéthylène (60) glycéryle trihydroxy stéarate conformément à la formule XVIII : 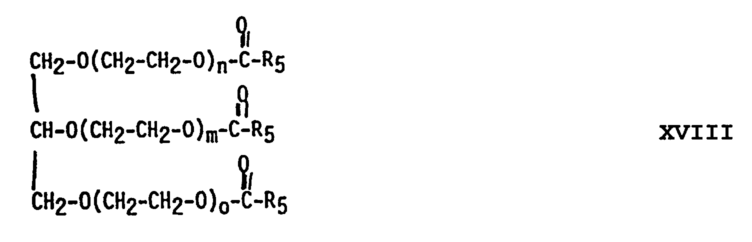 dans laquelle n+m+o vaut environ 60, et 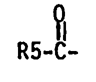 représente le radical 12-hydroxystéaryle,
est contenu en tant qu'agent stabilisant pour suspension et/ou en tant que lubrifiant pour valve.

19. Conditionnements pour gaz comprimés en aérosol en vue de l'administration par inhalation de substances biologiquement actives,
caractérisés en ce que
du polyoxyéthylène glycéryle triricinoléate conformément à la formule XIX : 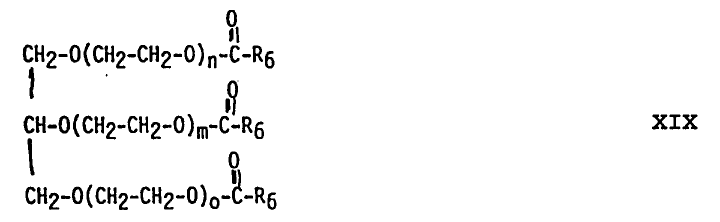 dans laquelle n+m+o vaut environ 35, et 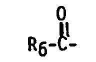 représente le reste d'acide ricinoléique,
est contenu en tant qu'agent stabilisant pour suspension et/ou en tant que lubrifiant pour valve.

20. Conditionnements pour gaz comprimés en aérosol conformément aux revendications 1 à 19,
caractérisés en ce que
la proportion de substances conformément aux formules I à XIX rapporté au poids total du mélange se situe entre 0,01 et 5 % en poids.

21. Conditionnement pour gaz comprimés en aérosol conformément aux revendications 1 à 19,
caractérisés en ce que
la proportion de composés des formules I à XIX rapporté au poids total du mélange, se situe entre 0,2 et 2,5 % en poids.

22. Conditionnement pour gaz comprimés en aérosol conformément aux revendications 1 à 19,
caractérisés en ce que
la proportion de composés des formules I à XIX rapporté au poids total du mélange, se situe entre 0,75 et 1,5 % en poids.

23. Conditionnement pour gaz comprimés en aérosol conformément aux revendications 1 à 22,
caractérisés en ce que
comme agent propulseur du TG 227 et/ou du TG 134a est contenu.

24. Procédé de production de conditionnements pour gaz comprimés en aérosol, appropriés pour l'administration par inhalation,
caractérisé en ce qu'
on utilise un agent stabilisant pour suspensions et/ou un lubrifiant pour valve conformément aux revendications 1 à 19.
